# EUROPEAN PATENT SPECIFICATION

(11) **EP 1 534 245 B1**
(45) Date of publication and mention of the grant of the patent: **16.07.2008**
(21) Application number: 03760665.4
(22) Date of filing: 20.06.2003
(51) Int. Cl.: A61K 9/20, A61K 31/42, A61K 31/43

(54) **RAPIDLY DISINTEGRATING TABLET**
SCHNELL ZERFALLENDE TABLETTE
COMPRIMES A DESAGREGATION RAPIDE

(30) Priority: 21.06.2002 SI 200200160
(43) Date of publication of application: 01.06.2005
(73) Proprietor: LEK Pharmaceuticals d.d., 61107 Ljubljana (SI)
(72) Inventor: SKULJ, Vesna, 1000 Ljubljana (SI); SIRCA, Judita, 1000 Ljubljana (SI); JENKO, Osel, Maja, 4000 Kranj (SI)
(74) Representative: Kröger, Bernd
(86) International application number: PCT/EP2003/006528
(87) International publication number: WO 2004/000281

(56) References cited:
- WO-A-00/66089
- WO-A-01/39746
- US-A- 5 985 823
- US-A- 6 117 451
- US-B1- 6 194 001
- US-B1- 6 200 604
- US-B1- 6 368 625
- US-B1- 6 391 335

## Description

The present invention belongs to the field of the pharmaceutical technology and it relates to rapidly disintegrating tablets intended to be used as orodispersible tablets or dispersible tablets which are dispersed in water prior to use.

Some patients, particularly pediatric and geriatric patients have difficulty swallowing or chewing solid dosage forms. Thus, there is a constant need for development of pharmaceutical formulations which rapidly disintegrate in the mouth of a patient and/or rapidly disperse in water.

Different techniques for preparation of rapidly disintegrating tablets are described in patent documents. The preparation of orodispersible tablets is generally technologically very demanding and expensive. Special, expensive manufacturing equipment is often needed. However, this formulation techniques appeared to be not applicable for the preparation of rapidly disintegrating tablets, containing a high amount of active ingredient. It is well known that for an effective treatment of diseases high doses of drugs and especially of antimicrobial compounds may have to be administered.

EP 910344 discloses fast-disintegrating and fast-dissolving compositions containing a high amount of drug. The active substance is incorporated in the granulate comprising water dispersible cellulose, which is microcrystalline cellulose and sodium carboxymethyl cellulose. The granulate is blended with a first and a second disintegrant, which is preferably selected from the group of superdisintegrants, and then compressed into tablets.

EP 80862 discloses water-dispersible composition of amoxicillin trihydrate and potassium clavulanate characterized in that it contains a non-hygroscopic watersoluble binder. Suitable binders are hydroxypropylmethyl cellulose, polyvinylpyrrolidone / polyvinyl acetate copolymer or hydroxypropylcellulose.

Dispersible tablets containing amoxicillin and clavulanic acid are described in WO 92/19227. These dispersible tablets are prepared as follows: The active substances are first roller-compacted together with a part of the excipients. The obtained granulate is then mixed with the remaining part of the excipients and the resulting mixture is compressed into tablets. Disintegrants used are the superdisintegrants such as, for example, cross-linked N-vinyl-2-pyrrolidone, croscarmellose sodium and/or sodium starch glycollate. In the description there is no data if the tablets are suitable to be used as orodispersible tablets.

WO 91/15197 discloses an effervescent formulation comprising amoxicillin trihydrat, potassium clavulanate and effervescent couple.

WO 00/66089 A, US-B1-6 391 335, US-B1-6 368 625 and US-B1-6 200 604 disclose orodispersible drug delivery systems comprising silicified microcrystalline cellulose.

US-A-6 117 451 and US-A-5 985 823 disclose rapid disintegrating tablets comprising silicified microcrystalline cellulose and an active agent.

The patent applications WO 96/21429, WO 97/17947 and WO 99/15155 describe the conventional pharmaceutical compositions which comprise silicified microcrystalline cellulose. These tablets also include a superdisintegrant, e.g., croscarmellose sodium.

Thus it is the object of the present invention to overcome the problems encountered in the prior art and to provide improved rapidly disintegrating tablets.

### Description of the invention

The above object is preferably achieved by a rapidly disintegrating tablet as specified in the claims.
The present invention thus relates to rapidly disintegrating tablets suitable to be used as orodispersible tablets which are disintegrated in the oral cavity, or (and) as dispersible tablets which are dispersed in water prior to ingestion. The tablets may be taken directly in the mouth or used dispersed in a glass of water.

The novel pharmaceutical formulation is patient-friendly since it enables to choose between the two modes of administration. It is particularly suitable for children and elderly individuals and for those who have difficulty in swallowing. An advantage of the pharmaceutical composition is that it may be used in circumstances when drinking water is not available.

The manufacturing process of the tablets is very simple, the addition of superdisintegrants is not required. This technology enables preparation of the orodispersible tablets with high dosage of the active substance.

The pharmaceutical formulation of the invention is especially appropriate for antibiotics which are usually administered in high doses. As the pharmaceutical formulation which is disintegrated in the mouth it is also suitable for use in pediatric patients in the age above 3 years, and as the pharmaceutical formulation which is dispersed in water it may be also used in pediatric patients in the age under 3 years. For pediatric use the tablets of the invention (one or more tablets) of different strengths may be administered to the child so that the dose is adjusted according to the child's body weight (and age), severity of the infection and the causative microorganism which is determined empirically or in the laboratory.

The present invention provides rapidly disintegrating tablets which contain amoxicillin and clavulanic acid, silicified microcrystalline cellulose in the amount of 30 to 95 % by weight of the tablet, a lubricant, a glidant, an antiadhesive, sweetening and flavouring agents.

The active substance is a combination of amoxicillin and clavulanic acid.

The dose of the active substance may vary depending on an individual active substance. The tablets may preferably contain up to 1500 mg of the active substance. The proportion of the active substances in the tablet is advantageously from 5 to 70% by weight of the tablet.

Silicified microcrystalline cellulose may be any commercially available form of this ingredient, for example, Prosolv SMCC, described in WO 96/21429 and manufactured by Penwest Company. There are different grades of silicified microcrystalline cellulose available. The amount of silicified microcrystalline cellulose in the tablets of the invention is about 30 to about 95 % by weight of the tablets. A ratio of the active substance and silicified microcrystalline cellulose may preferably be in the range 0.5 : 1 to 2.5 :1.

A lubricant is preferably selected from the group of hydrophobic lubricants such as hydrogenated fatty oils, magnesium stearate, and stearic acid. Especially suitable lubricant is selected from hydrogenated vegetable oils. Preferable lubricant is hydrogenated castor oil Cutina HR, Henkel.
Sweetening agents used in the tablets may be artificial sweetening agents such as, for example aspartame, saccharin sodium, acesulfame potassium or also natural sugars. Flavouring agents may preferably be selected from conventional flavours such as natural flavouring agents, nature-identical flavouring agents, and artificial flavouring agents of different tastes.

The tablets of the present invention further include antiadhesives such as, for example, talc, glidants such as, for example, colloidal silicon dioxide, Aerosil 200.

If necessary, a particularly unpleasant taste of the active substances may be previously masked.

The process for preparation of the tablets of this invention is very simple. The active substance and excipients are blended; the mixture is homogenized, sieved and directly formed into tablets, preferably by compressing. Previous dry or wet granulation is not needed.

The tablets of the invention correspond to all pharmacopoeial standards for tablets, orodispersible tablets and dispersible tablets. The tablets are of the pleasant taste and rapidly disintegrate in the mouth or disperse in water. The physical characteristics of the tablets are suitable for packaging on a conventional packaging line which with rapidly disintegrating tablets manufactured by other technologies is not conventional.

The tablets of this invention may optionally be coated with a sufficiently thin and water soluble coating layer which does no influence on the ability of the tablet to disintegrate rapidly in the mouth. Suitable coating materials include disaccharides such as sucrose, polysaccharides such as maltodextrins and pectin, and cellulose derivatives such as hydroxypropyl cellulose and hydroxypropylmethyl cellulose.

The combination of the antibiotic amoxicillin and clavulanic acid, an inhibitor of beta-lactamase, is the well recognized and widely used medicament for treating bacterial infections in adult and pediatric patients. It is available in several dosage forms such as, for example, conventional tablets, chewable tablets, sachets, powder for preparation of oral suspension, dispersible tablets and controlled-release tablets.

The use of silicified microcrystalline cellulose has enabled manufacture of high dosage amoxicillin / clavulanic acid orodispersible and dispersible tablets with simple composition and technology.

Amoxicillin may be in the form of trihydrate or as crystalline sodium amoxicillin, clavulanic acid may be in the form of a salt, preferably potassium clavulanate. The ratio of amoxicillin and clavulanic acid is preferably from 2:1 to 30:1, especially suitable are the ratios 4:1, 7:1, 8:1, 12:1, 14:1 and 16:1.

The tablets may preferably contain from 250 to 1500 mg of amoxicillin and the appropriate amount of clavulanic acid. They may be prepared, for example, as tablets 250/125, 500/125, 500/62.5, 875/125, 1000/125, 1000/62.5, 400/57, 200/28.5, 250/62.5, 125/31.3. The weights being expressed as free parent acids amoxicillin and clavulanic acid.

The proportion of silicified microcrystalline cellulose in the tablet is from 30% to 90% by weight of the tablet. Preferably SMCC 90 is used.

The lubricant may be any lubricant from the group of hydrophobic lubricants. Especially suitable is hydrogenated castor oil Cutina HR, Henkel.

Conventional sweetening and flavouring agents are added. The most convenient sweetening agents are aspartame and saccharin sodium. As the flavouring agent a combination of two flavours such as tropical blend and orange is favourable.

The tablets also contain other excipients such as glidants such as colloidal silicon dioxide, and antiadhesives.

Generally the tablets which contain amoxicillin and clavulanic acid cannot be prepared by direct compression method. Usually one or both active substances and a part of excipients are pre-granulated by dry granulation process such as slugging or roller compaction. The granulate may then be mixed with the remaining part of the excipients and then the mixture is compressed into tablets. The tablets of the present invention are prepared according to the simple process by direct compression into tablets. Previous granulation is not necessary. All ingredients are blended, homogenized, sieved and directly formed, preferably compressed into tablets. As potassium clavulanate is highly moisture sensitive, previously dried ingredients should be used. The manufacturing of the tablets of the invention is preferably carried out under conditions of relative humidity not exceeding 25% RH, more suitably less than 20% RH.

Rapidly disintegrated tablets containing amoxicillin and clavulanic acid are also suitable for use in pediatric patients. Since they are of the pleasant taste and are simply ingested (dispersed directly in the mouth), they are suitable for children above 3 years old, and as the pharmaceutical formulation which is dispersed in water it may be also used in pediatric patients in the age under 3 years. The dosage should be adjusted according to child's body weight (and age), and severity of the infection and the causative microorganism determined empirically or in the laboratory.

The technology of preparation of the tablets of the invention provides formulating the tablets containing different unit doses of the active substance thus enabling the use of an appropriate dosage by administering the tablets of different strengths aimed at attaining the optimal dosage regarding the child's body weight (and age), severity of the infection and causative microorganism.

The tablets may replace the existing pediatric suspension formulations comprising amoxicillin and clavulanic acid.

For example, tablets containing 400 mg of amoxicillin and 57 mg of clavulanic acid may be prepared. One such tablet may replace the 5 ml of the existing pediatric suspension 400/57 for twice daily administration. Likewise, the tablet containing 200 mg of amoxicillin and 28.5 mg of clavulanic acid can replace the 5 ml of the existing pediatric suspension 200/28.5. One tablet containing 250 mg of amoxicillin and 62.5 mg of clavulanic acid can replace the 5 ml of existing pediatric suspension 250/62.5 per 5 ml. Likewise, the tablet containing 125 mg of amoxicillin and 31.25 mg of clavulanic acid can replace the 5 ml of the existing pediatric suspension 125/31.25 per 5 ml. The dosage in pediatric population with these tablets can be adjusted by taking a half of a tablet or a quarter of a tablet like in the case of suspensions where adjustments are possible by taking appropriate volumes of appropriate suspensions.
The total daily dosage depends on the weight (and age) of a child, on the suspected microorganism causing the infection and on the severity of the infection.

The present invention is illustrated but in no way limited by the following examples:

### Example 1:

### Composition of one tablet:

| INGREDIENTS | | |
|---|---|---|
| Amoxicillin (in the form of trihydrate) | 875 | mg |
| Clavulanic acid (in the form of potassium clavulanate) | 125 | mg |
| Aspartame | 9 | mg |
| Flavour | 36 | mg |
| Aerosil 200 | 18 | mg |
| Cutina HR | 36 | mg |
| Talc | 18 | mg |
| Prosolv SMCC 90 | to 1932 | mg |

The method of manufacture:
All ingredients are blended, homogenized, sieved and compressed directly into tablets.

### Example 2:

### Composition of one tablet:

| INGREDIENTS | | |
|---|---|---|
| Amoxicillin (in the form of trihydrate) | 500 | mg |
| Clavulanic acid (in the form of potassium clavulanate) | 125 | mg |
| Aspartame | 6.5 | mg |
| Flavour | 26 | mg |
| Aerosil 200 | 13 | mg |
| Cutina HR | 26 | mg |
| Talc | 13 | mg |
| Prosolv SMCC 90 | to 1300 | mg |

All ingredients are blended, homogenized, sieved and compressed directly into tablets.

### Example 3:

### Composition of one tablet:

| INGREDIENTS | | |
|---|---|---|
| Amoxicillin (in the form of trihydrate) | 437.5 | mg |
| Clavulanic acid (in the form of potassium clavulanate) | 62.5 | mg |
| Aspartame | 4.5 | mg |
| Flavour | 18 | mg |
| Aerosil 200 | 9 | mg |
| Cutina HR | 18 | mg |
| Talc | 9 | mg |
| Prosolv SMCC 90 | to 966 | mg |

All ingredients are blended, homogenized, sieved and compressed directly into tablets.

### Example 4:

### Composition of one tablet:

| INGREDIENTS | | |
|---|---|---|
| Amoxicillin (in the form of trihydrate) | 250 | mg |
| Clavulanic acid (in the form of potassium clavulanate) | 62.5 | mg |
| Aspartame | 3.25 | mg |
| Flavour | 13 | mg |
| Aerosil 200 | 6.5 | mg |
| Cutina HR | 13 | mg |
| Talc | 6.5 | mg |
| Prosolv SMCC 90 | to 650 | mg |

All ingredients are blended, homogenized, sieved and compressed directly into tablets.

### Example 5:

### Composition of one tablet:

| INGREDIENTS | | |
|---|---|---|
| Amoxicillin (in the form of trihydrate) | 1000 | mg |
| Clavulanic acid (in the form of potassium clavulanate) | 125 | mg |
| Aspartame | 10.1 | mg |
| Flavour | 40.5 | mg |
| Aerosil 200 | 20.3 | mg |
| Cutina HR | 40.5 | mg |
| Talc | 20.3 | mg |
| Prosolv SMCC 90 | to 2174 | mg |

All ingredients are blended, homogenized, sieved and compressed directly into tablets.

### Example 6:

### Composition of one tablet:

| INGREDIENTS | | |
|---|---|---|
| Amoxicillin (in the form of trihydrate) | 400 | mg |
| Clavulanic acid (in the form of potassium clavulanate) | 57 | mg |
| Aspartame | 4.7 | mg |
| Flavour | 19 | mg |
| Aerosil 200 | 9.5 | mg |
| Cutina HR | 19 | mg |
| Talc | 9.5 | mg |
| Prosolv SMCC 90 | to 950 | mg |

All ingredients are blended, homogenized, sieved and compressed directly into tablets.

## Claims

1. An orodispersible or dispersible tablet containing:
- amoxicillin and clavulanic acid,
- silicified microcrystalline cellulose in the amount of 30 to 95% by weight of the tablet,
- a lubricant, a glidant, an antiadhesive, sweetening and flavouring agents.

2. The tablet according to claim 1, wherein the amoxicillin is in the form of amoxicillin trihydrate.

3. The tablet according to claim 1, wherein the clavulanic acid is in the form of potassium clavulanate.

4. The tablet according to any of claim 1 to 3, wherein the ratio of amoxicillin to clavulanic acid is in the range of 2:1 to 30:1.

5. The tablet according to any of claims 1 to 3, wherein the ratio of amoxicillin to clavulanic acid is 4:1.

6. The tablet according to any of claims 1 to 3, wherein the ratio of amoxicillin to clavulanic acid is 7:1.

7. The tablet according to claim 1, wherein the proportion of the active substance in the tablet is 5 to 70% by weight of the tablet.

8. The tablet according to claim 1, wherein the ratio of amoxicillin and clavulanic acid to the silicified microcrystalline cellulose is 0.5:1 to 2.5:1.

9. The tablet according to claim 1 comprising 875 mg of amoxicillin and 125 mg of clavulanic acid.

10. The tablet according to claim 1 comprising 500 mg of amoxicillin and 125 mg of clavulanic acid.

11. The tablet according to claim 1, wherein hydrogenated castor oil is contained as a lubricant.

12. A process for the manufacture of a tablet according to claim 1 comprising the steps of:
- blending amoxicillin, clavulanic acid, silicified microcrystalline cellulose as the disintegrant and optional exicipients;
- homogenizing the obtained mixture;
- sieving the homogenized mixture; and
- forming tablets therefrom.

## Patentansprüche

1. Orodispergierbare oder dispergierbare Tablette, die enthält
- Amoxicillin und Clavulansäure,
- silizifizierte mikrokristalline Cellulose in einer Menge von 30 bis 95 Gew.-% der Tablette, und
- ein Schmiermittel, ein Gleitmittel, ein antiadhäsives Mittel, Süßungsmittel und Aromastoffe.

2. Tablette nach Anspruch 1, worin das Amoxicillin in der Form von Amoxicillintrihydrat vorliegt.

3. Tablette nach Anspruch 1, worin die Clavulansäure in der Form von Kaliumclavulanat vorliegt.

4. Tablette nach einem der Ansprüche 1 bis 3, worin das Verhältnis von Amoxicillin zu Clavulansäure im Bereich von 2:1 bis 30:1 liegt.

5. Tablette nach einem der Ansprüche 1 bis 3, worin das Verhältnis von Amoxicillin zu Clavulansäure 4:1 beträgt.

6. Tablette nach einem der Ansprüche 1 bis 3, worin das Verhältnis von Amoxicillin zu Clavulansäure 7:1 beträgt.

7. Tablette nach Anspruch 1, worin das Verhältnis des Wirkstoffs in der Tablette 5 bis 70 Gew.-% der Tablette beträgt.

8. Tablette nach Anspruch 1, worin das Verhältnis von Amoxicillin und Clavulansäure zur silizifizierten mikrokristallinen Cellulose 0,5:1 bis 2,5:1 beträgt.

9. Tablette nach Anspruch 1, umfassend 875 mg Amoxicillin und 125 mg Clavulansäure.

10. Tablette nach Anspruch 1, umfassend 500 mg Amoxicillin und 125 mg Clavulansäure.

11. Tablette nach Anspruch 1, worin hydriertes Rizinusöl als Schmiermittel enthalten ist.

12. Verfahren zur Herstellung einer Tablette nach Anspruch 1, umfassend die folgenden Stufen:
- Vermischung von Amoxicillin, Clavulansäure, silizifizierter mikrokristalliner Cellulose als das Zerfallhilfsmittel und optional Exzipientien,
- Homogenisation des erhaltenen Gemisches,
- Siebung des homogenisierten Gemisches, und
- Formung von Tabletten daraus.

## Revendications

1. Comprimé orodispersible ou dispersible comprenant :
- de l'amoxicilline et de l'acide clavulanique,
- de la cellulose microcristalline silicifiée à raison de 30 à 95 % en poids du comprimé,
- un lubrifiant, un agent glissant, un agent anti-adhérant, un édulcorant et un aromatisant.

2. Comprimé selon la revendication 1, dans lequel l'amoxicilline se présente sous forme d'amoxicilline trihydrate.

3. Comprimé selon la revendication 1, dans lequel l'acide clavulanique se présente sous forme de clavulanate de potassium.

4. Comprimé selon l'une quelconque des revendications 1 à 3, dans lequel le rapport entre amoxicilline et acide clavulanique est compris dans la plage de 2:1 à 30:1.

5. Comprimé selon l'une quelconque des revendications 1 à 3, dans lequel le rapport entre amoxicilline et acide clavulanique est de 4:1.

6. Comprimé selon l'une quelconque des revendications 1 à 3, dans lequel le rapport entre amoxicilline et acide clavulanique est de 7:1.

7. Comprimé selon la revendication 1, dans lequel la proportion de substance active dans le comprimé est comprise entre 5 et 70 % en poids du comprimé.

8. Comprimé selon la revendication 1, dans lequel le rapport entre amoxicilline plus acide clavulanique et cellulose microcristalline silicifiée est de 0,5:1 à 2,5:1.

9. Comprimé selon la revendication 1, comprenant 875 mg d'amoxicilline et 125 mg d'acide clavulanique.

10. Comprimé selon la revendication 1, comprenant 500 mg d'amoxicilline et 125 mg d'acide clavulanique.

11. Comprimé selon la revendication 1, comprenant de l'huile de ricin hydrogénée en tant que lubrifiant.

12. Procédé de fabrication d'un comprimé selon la revendication 1, comprenant les étapes consistant à :
- mélanger de l'amoxicilline, de l'acide clavulanique et de la cellulose microcristalline silicifiée en tant qu'agent désintégrant et des excipients facultatifs ;
- homogénéiser le mélange obtenu ;
- tamiser le mélange homogénéisé ; et
- former des comprimés à partir de celui-ci.
